# EUROPÄISCHE PATENTSCHRIFT

(11) **EP 0 296 097 B1**
(45) Veröffentlichungstag und Bekanntmachung des Hinweises auf die Patenterteilung: **15.09.1993**
(21) Anmeldenummer: 88730135.6
(22) Anmeldetag: 14.06.1988
(51) Int. Cl.: A61K 31/565

(54) **Arzneimittel enthaltend Progesteronsynthesehemmer vom Typ des Trilostans oder Epostans und Antigestagene**
Medicine containing inhibitors of the progesterone synthesis of the trilostane or epostane type and antigestagens
Médicament contenant des inhibiteurs de synthèse de la progestérone du type trilostane ou épostane et des antigestagènes

(30) Priorität: 16.06.1987 DE 3720420
(43) Veröffentlichungstag der Anmeldung: 21.12.1988
(73) Patentinhaber: SCHERING AKTIENGESELLSCHAFT, 13342 Berlin (DE)
(72) Erfinder: Elger, Walter, Dr., D-1000 Berlin 33 (DE); Beier, Sybille, Dr., D-1000 Berlin 31 (DE); Kosub, Beate, D-1000 Berlin 37 (DE); Fähnrich, Marianne, D-1000 Berlin 51 (DE); Chwalisz, Krzysztof, Dr., D-1000 Berlin 45 (DE); Hasan, Syed Hamiduddin, Dr., D-1000 Berlin 19 (DE); Potts, Gordon Oliver, Dr., North Chatham New York 12132 (US)

(56) Entgegenhaltungen:
- US-A- 4 062 954
- US-A- 4 670 426
- CHEMICAL ABSTRACTS, Band 101, 1984, Seite 80, Zusammenfassung 66239v, Columbus, Ohio, US B.W. SNYDER et al.: "Inhibition of ovulation in rats with epostane, an inhibitor of 3beta-hydroxysteroid dehydrogenase"
- J. steroid Biochem., vol. 31, no. 1, pp. 137-146, 1988; M.A. Shaw et al.

## Beschreibung

Die Erfindung betrifft ein Arzneimittel, welches einen Progesteronsynthesehemmer (ProH) vom Typ des Trilostans oder Epostans und ein Antigestagen (AG) enthält.

Progesteronsynthesehemmer (ProH) vom Typ des Trilostans oder Epostans und Antigestagene (AG) können zur gemeinsamen Verwendung für die Einleitung der Geburt zum Termin bei Mensch und Tier und für den Abbruch der normalen oder pathologischen Gravidität dienen.

Weiter ist die erfindungsgemäße Kombination aus Progesteronsynthesehemmer und Antigestagen zur Behandlung hormonabhängiger Tumore, der Endometriose und der Dysmenorrhoe geeignet.

Um Gefahren für Mutter und/oder Kind abzuwenden, ist es manchmal erforderlich, eine Geburt künstlich einzuleiten oder eine Schwangerschaft vorzeitig zu beenden. Dafür stehen chirurgische Techniken und pharmakologische Methoden zur Verfügung.

Eine mögliche pharmakologische Methode ist die vaginale oder intramuskuläre Applikation von Prostaglandinen, die im Falle des Schwangerschaftsabbruchs im 1. oder 2. Schwangerschaftstrimester (Contraception 1983, Vol. 27, 51-60 und Int. J. Gynaecol. Obstet. 1982, Vol. 20, 383-386) vorgenommen wird.

Der Vorteil der Prostaglandin-Anwendung ist ihre Einsatzmöglichkeit über einen langen Zeitraum der Schwangerschaft. Als Nachteile der Prostaglandine sind akute Nebenwirkungen wie Schmerzen und Übelkeit zu nennen. Außerdem liegt die Erfolgsrate im Falle des Schwangerschaftsabbruchs in fortgeschrittenen Phasen der Schwangerschaft auch bei einer längeren Dauer der Prostaglandinbehandlung nicht über 90 %.

Eine andere Möglichkeit der Beendigung einer Schwangerschaft besteht in der Applikation eines Antigestagens (Med. et Hyg. 1982, Vol. 40, 2087-2093). Antigestagene sind besser verträglich als Prostaglandine, haben aber eine geringere Wirksamkeit, eine größere Latenz und individuelle Variabilität des Wirkungseintritts im Vergleich mit den Prostaglandinen. Außerdem wurde in der Klinik beobachtet, daß sie eine Tendenz zu z.T. schweren Blutungen aufweisen.

Die gemeinsame Verwendung von Prostaglandinen und Antigestagenen (EP 84730108.2) bringt zwar unbestreitbare Vorteile gegenüber der alleinigen Applikation der einzelnen Wirkstoffe (vor allem Reduktion der jeweiligen Wirkstoffmenge), löst aber z.B. nicht die Probleme, die generell bei der Verwendung von Prostaglandinen auftreten: unerwünschte Nebenwirkungen wie gastrointestinale Effekte oder Uterus-Schmerzen, die Behandlung muß stationär erfolgen, Lagerung und Haltbarkeit des Arzneimittels sind aufgrund mangelnder Stabilität begrenzt und/oder aufwendig, die anwenderfreundlichste Applikationsform, nämlich die orale, ist nicht möglich und damit auch nicht die Kombination beider Wirkstoffe in einer Tablette, Pille oder einem Dragee.

Als eine weitere Möglichkeit, eine Schwangerschaft vorzeitig zu beenden, ist die Behandlung mit Progesteronsynthesehemmern wie Epostan diskutiert worden (US-Patent 4,160,027). Es zeigte sich jedoch, daß beim Meerschweinchen in der fortgeschrittenen Gravidität auch mit solch hohen Dosen, die den Serumprogesteronspiegel deutlich absinken lassen, kein Abort ausgelöst werden kann. In der Frühgravidität des Menschen erhält man auch mit höchsten Epostan-Dosen keine 100 %ige Erfolgsrate.

Erst wieder in der Kombination mit Prostaglandinen ist die klinische Behandlung erfolgreich, wobei jedoch sehr hohe Wirkstoffmengen (30 mg PGE₂, 5 x 600 mg Epostan) benötigt werden. Neben diesem Nachteil ist die Methode mit der oben diskutierten Problematik der Verwendung von Prostaglandinen belastet.

Pharmazeutische Zusammensetzungen zur post-coitalen Fertilitätskontrolle, die einen kompetitiven Progesteronantagonisten (Antigestagen) sowie einen Progesteron- und Östrogensyntheseblocker enthalten, sind bereits im US-Patent 4,670,426 beschrieben. Als typische Vertreter für den zu verwendenden kompetitiven Progesteronantagonisten sind Fluocinolonacetonid, Triamcinolonacetonid, Steroide mit einem zyklischen 16,17-Acetat mit Aceton und 17β-Hydroxy-11β-(4-dimethylaminophenyl-1)-17α-prop-1-inyl)-estra-4,9-dien-3-on und äquivalente Derivate erwähnt. Der typische Gehalt liegt dabei zwischen 20 und 50 mg. Als Beispiele für den Progesteron- und Östrogensyntheseblocker sind Aminoglutethimid, 2α-Cyano-4,4,17α-trimethyl-5-androst-5-en-17β-ol-3-on, 20,25-Diazocholesterol und Verbindungen mit äquivalenter Aktivität angeführt, und zwar in einer Dosis von 300 bis 1000 mg. Die Anwendung der Zusammensetzung hat gemäß US-Patent 4,670,426 möglichst früh innerhalb der ersten Woche nach dem Geschlechtsverkehr über einen Zeitraum von 3 Tagen zu erfolgen; am besten sollte die Behandlung 2 bis 6 Tage fortgesetzt werden. Die Verhinderung der Nidation und somit einer Schwangerschaft wird durch den synergistischen Effekt bei der gemeinsamen Anwendung der beiden Bestandteile der Zusammensetzung bewirkt, und zwar mit einer Erfolgsrate in der Größenordnung von 90 % oder mehr.

In neuerer Zeit wird auch die Verwendung von Antigestagenen im Bereich der Tumortherapie, insbesondere für die Indikation Mammacarcinom diskutiert. Eine erste Phase-II-Studie mit dem bereits erwähnten 17β-Hydroxy-11β-(4-dimethylaminophenyl)-17β-prop-1-inyl)-estra-4,9-dien-3-on an postmenopausalen bzw. ovariektomierten Endokrintherapieresistenten Patientinnen mit metastasierendem Mammacarcinom wird von Maudelonde et al. in Hormonal Manipulation of Cancer, Eds. J.G.M. Klijn, R. Paridaens und J.A. Folkens in Raven Press, S. 55 (1987) berichtet.

Der Erfindung liegt somit die Aufgabe zugrunde, Arzneimittel für die angegebenen Verwendungen bereitzustellen, die die oben genannten Nachteile nicht aufweisen, gleichzeitig eine hohe, möglichst höhere Wirksamkeit im Vergleich zu den bekannten Mitteln haben und weniger Nebenwirkungen als diese aufweisen.

Diese Aufgabe wird durch die Erfindung gelöst.

Es wurde gefunden, daß die Verwendung einer Kombination von Progesteronsynthesehemmstoffen und Antigestagenen (kompetitiven Progesteronantagonisten), d.h. von Substanzen, die beide als Wirkungsprinzip die Hemmung der Progesteronwirkung aufweisen und jede für sich genommen auch in hohen Dosierungen nur unvollständig oder gar nicht wirksam sind, selbst bei dramatischer Reduktion dieser Dosen überraschenderweise voll wirksam sind.

Eine Wirksamkeit von 100 % ist unbedingt erforderlich, da eine fruchtschädigende Wirkung der beteiligten Verbindungen praktisch nie ganz sicher ausgeschlossen werden kann.

Die erfindungsgemäße Kombination ist dabei nicht nur in hohem Maße zur Geburtseinleitung und zum Abbruch der normalen oder pathologischen Gravidität geeignet; sie findet zusätzliche Verwendung als Mittel gegen Endometriose, Dysmenorrhoe und zur Behandlung von hormonabhängigen Tumoren, wie zum Beispiel Mammacarcinom und Meningeom.

Bei der Behandlung homonabhängiger Tumore erweist sich das erfindungsgemäße Mittel der alleinigen Verwendung von Antigestagenen als überlegen.

Progesteronsynthesehemmer vom Typ des Trilostans oder Epostans und Antigestagen werden gemeinsam oder getrennt, gleichzeitig und/oder zeitlich abgestuft (sequential), in einem Gewichtsverhältnis von im wesentlichen 1:40 bis 60:1, vorzugsweise 1:30 bis 30:1, verwendet.

Die gemeinsame Behandlung mit Progesteronsynthesehemmern und Antigestagenen erfolgt in der Regel über 1 bis 4, vorzugsweise 1 bis 2 Tage.

Vorzugsweise können Progesteronsynthesehemmer und Antigestagen auch kombiniert in einer Dosiseinheit appliziert werden.

Als Antigestagene kommen alle Verbindungen infrage, die eine starke Affinität zum Gestagenrezeptor (Progesteronrezeptor) besitzen und dabei höchstenfalls geringe eigene gestagene Aktivität zeigen. Als kompetitive Progesteronantagonisten kommen beispielsweise folgende Steroide infrage:
11β-[(4-N,N-Dimethylamino)-phenyl]-17β-hydroxy-17α-propinyl-4,9(10)-estradien-3-on (RU 486),
11β-[(4-N,N-Dimethylamino)-phenyl]-17β-hydroxy-18-methyl-17α-propinyl-4,9(10)-estradien-3-on und
11β-[(4-N,N-Dimethylamino)-phenyl]-17aβ-hydroxy-17aα-propinyl-D-homo-4,9(10)-16-estratrien-3-on (Europäische Patentanmeldung 82400025.1 - Veröffentlichungs-Nr. 0 057 115);
ferner
11β-p-Methoxyphenyl-17β-hydroxy-17α-ethinyl-4,9(10)-estradien-3-on (Steroids 37 (1981) 361-382) und
11β-(4-Dimethylaminophenyl)-17α-hydroxy-17β-(3-hydroxypropyl)-13α-methyl-4,9-gonadien-3-on (Europäisches Patent 0 129 499).

Die Antigestagene werden gemäß vorliegender Erfindung in Mengen eingesetzt, die unterhalb der sonst für den Schwangerschaftsabbruch üblichen (und für eine 100 %ige Erfolgsrate trotzdem oft nicht ausreichenden) Mengen liegen. Im allgemeinen wird man mit 5 bis 200 mg 11β-[(4-N,N-Dimethylamino)-phenyl]-17β-hydroxy-17α-propinyl-4,9(10)-estradien-3-on pro Tag oder einer biologisch äquivalenten Menge eines anderen Antigestagens auskommen.

Für die gemäß der vorliegenden Erfindung zu verwendenden Progesteronsynthesehemmer kommen alle den Progesteronspiegel im Blut senkenden Inhibitoren des 3β-Hydroxysteroid-Dehydrogenase-Enzymsystems, insbesondere vom Typ des Trilostans (4α,5-Epoxy-3,17β-dihydroxy-5α-androst-2-en-2-carbonitril) und Epostans (4α,5-Epoxy-3,17β-dihydroxy-4β,17α-dimethyl-5α-androst-2-en-2-carbonitril) infrage. Entscheidend ist, daß die zu verwendenden Progesteronsynthesehemmer möglichst selektiv wirken, d.h. im wesentlichen nur den Spiegel des Progesterons im Blut nicht aber die gonadale Steroidhormonsekretion senken.

Als Verbindungen vom Typ des Trilostans und/oder Epostans sind unter anderem 5-Androstane, die die nachstehend genannten Strukturmerkmale aufweisen, zu verstehen: 2-Cyano-4α,5α-epoxy und 17β-hydroxy oder -alkanoyloxy (mit 1 - 6 C-Atomen), vgl. US-Patent 4,160,027. Zu einer besonders bevorzugten Variante gehören solche 5α-Androstane, die anstelle der 3-Oxogruppe die Struktureinheit 2-En-3-hydroxy enthalten. Zusätzlich können alle diese Verbindungen Substituenten tragen, beispielsweise die im US-Patent 4,160,027 beschriebenen.

Die Progesteronsynthesehemmer werden in Mengen eingesetzt, die weit unterhalb der sonst für die angegebenen Indikationen üblichen Mengen liegen. Bei der Verwendung von Epostan als Progesteronsynthesehemmer wird man in der Regel mit insgesamt 5 - 600 mg, vorzugsweise 30 - 300 mg, auskommen. Eine Dosiseinheit enthält etwa 5 - 300 mg Epostan oder eine biologisch äquivalente Menge eines anderen Progesteronsynthesehemmers.

Die Antigestagene und Progesteronsynthesehemmer können zum Beispiel lokal, topisch, enteral oder parenteral appliziert werden.

Für die bevorzugte orale Applikation kommen insbesondere Tabletten, Dragees, Kapseln, Pillen, Suspensionen oder Lösungen infrage, die in üblicher Weise mit den in der Galenik üblichen Zusätzen und Trägersubstanzen hergestellt werden können. Für die lokale oder topische Anwendung kommen beispielsweise Vaginalzäpfchen oder transdermale Systeme wie Hautpflaster infrage.

Gemäß der vorliegenden Erfindung wird zum Abbruch der Gravidität und zur Einleitung der Geburt die Kombination aus Antigestagen und Progesteronsynthesehemmer in jedem Fall nach der Nidation appliziert, vorzugsweise im zweiten oder dritten Drittel der Gravidität, im Fall der Geburtseinleitung kurz bevor oder am Geburtstermin. Die Verbindungen können in jeder Reihenfolge, vorzugsweise gleichzeitig, gegeben werden. Im Fall der sequentiellen Gabe kann die als zweite gegebene Verbindung zu jeder Zeit nach Gabe der zuerst applizierten Verbindung gegeben werden, solange sie noch in der Patientin gleichzeitig mit einer wirksamen Menge der zuerst applizierten Verbindung bioverfügbar wird. Beispielsweise kann der Progesteronsynthesehemmer innerhalb von 1 bis 2 Tagen nach dem Antigestagen gegeben werden.

Eine Dosiseinheit enthält etwa 10 bis 200 mg 11β-[(4-N,N-Dimethylamino)-phenyl]-17β-hydroxy-17α-propinyl-4,9(10)-estradien-3-on oder eine biologisch äquivalente Menge eines anderen Antigestagens.

Die nachstehenden Beispiele sollen die galenische Formulierung erläutern.

### Beispiel 1

| **Zusammensetzung einer Tablette mit 11β-[(4-N,N-Dimethylamino)-phenyl]-17β-hydroxy-17α-propinyl-4,9(10)-estradien-3-on zur oralen Applikation** | |
|---|---|
| 10,0 mg | 11β-[(4-N,N-Dimethylamino)-phenyl]-17β-hydroxy-17α-propinyl-4,9(10)-estradien-3-on |
| 140,5 mg | Laktose |
| 69,5 mg | Maisstärke |
| 2,5 mg | Polyvinylpyrrolidon 25 |
| 2,0 mg | Aerosil |
| 0,5 mg | Magnesiumstearat |
| 2̅2̅5̅,̅0̅ m̅g̅ | Gesamtgewicht |

### Beispiel 2

| **Zusammensetzung einer Tablette mit 4α,5-Epoxy-3,17β-dihydroxy-4β,17α-dimethyl-5α-androst-2-en-2-carbonitril (Epostan) zur oralen Applikation** | |
|---|---|
| 30,0 mg | 4α,5-Epoxy-3,17β-dihydroxy-4β,17α-dimethyl-5α-androst-2-en-2-carbonitril |
| 201,0 mg | Laktose |
| 139,0 mg | Maisstärke |
| 5,0 mg | Polyvinylpyrrolidon 25 |
| 4,0 mg | Aerosil |
| 1,0 mg | Magnesiumstearat |
| 3̅8̅0̅,̅0̅ m̅g̅ | Gesamtgewicht |

### Beispiel 3

| **Zusammensetzung einer Tablette mit 4α,5-Epoxy-3,17β-dihydroxy-5α-androst-2-en-2-carbonitril (Trilostan) zur oralen Applikation** | |
|---|---|
| 20,0 mg | 4α,5-Epoxy-3,17β-dihydroxy-5α-androst-2-en-2-carbonitril |
| 281,0 mg | Laktose |
| 139,0 mg | Maisstärke |
| 5,0 mg | Polyvinylpyrrolidon 25 |
| 4,0 mg | Aerosil |
| 1,0 mg | Magnesiumstearat |
| 450,0 mg | Gesamtgewicht |

### Pharmakologische Beobachtungen

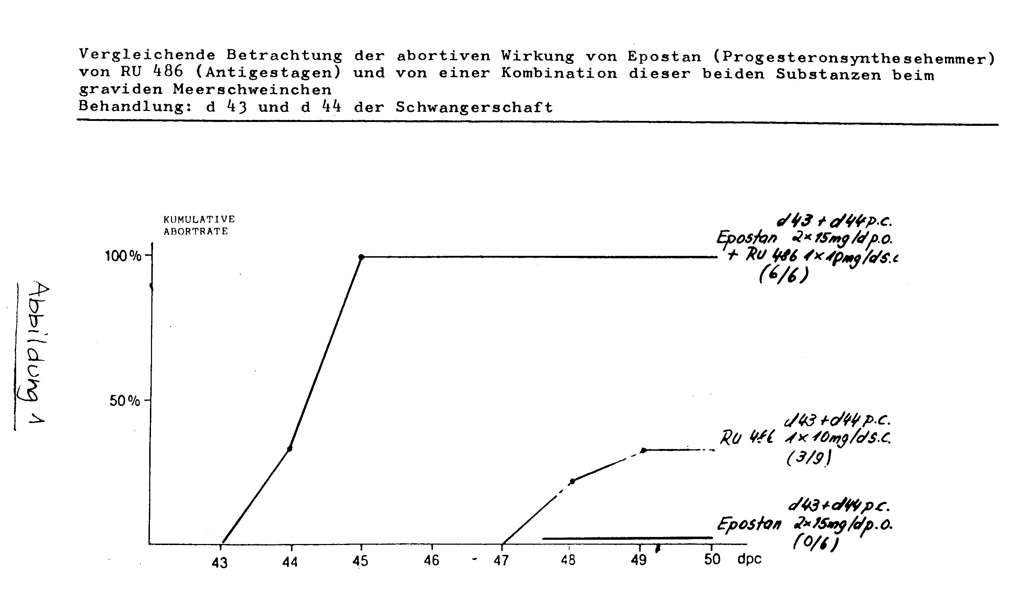

Für die Versuche an graviden Meerschweinchen wurden der Progesteronsynthesehemmer Epostan und das Antigestagen 11β-[(4-N,N-Dimethylamino)-phenyl]-17β-hydroxy-17α-propinyl-4,9(10)-estradien-3-on (RU 486) als Modellsubstanzen ausgewählt. Die geprüften Dosierungen sind der Abbildung zu entnehmen.

### Untersuchungen an graviden Meerschweinchen

### Prüfung der Kombination

### Versuchsbeschreibung

Gravide Meerschweinchen mit einem Körpergewicht von ca. 800 g wurden am 42. Tag der Schwangerschaft in den Versuch genommen (der 2. Tag der Vaginalöffnung in der Anpaarungsphase wurde als erster Tag der Gravidität gerechnet). Vor Versuchsbeginn wurde die Gravidität durch Palpation kontrolliert. Die Behandlung mit den ausgewählten Prüfsubstanzen bzw. der Kombination erfolgte durch tägliche orale (Epostan) bzw. subcutane (RU 486) Applikation am 43. und 44. Tag der Gravidität. Das Antigestagen wurde dazu in Benzylbenzoat + Rizinusöl (Mischungsverhältnis 2 + 4,5) gelöst und die tägliche Dosis in einem Volumen von 1,0 ml s.c. injiziert. Die tägliche Dosis Epostan wurde mikrokristallin suspendiert in Myrj^{(R)}/Kochsalzlösung verteilt auf zwei gleiche Dosen morgens und nachmittags appliziert. Das eventuelle Ausstoßen von Früchten wurde unter und nach der Behandlung mehrfach täglich kontrolliert. Am 50. Tag der Gravidität wurden die Tiere getötet. Die Uteri wurden inspiziert und die Foeten befundet.

### Ergebnisse

Die Ergebnisse der Versuche zur Abortinduktion beim graviden Meerschweinchen bei kombinierter Verabreichung von Antigestagenen und Progesteronsynthesehemmer sind der Abbildung zu entnehmen.

### Progesteronsynthesehemmer

Mit einer Dosis von 30,0 mg/Tag p.o. war Epostan total inaktiv im Hinblick auf abortive Wirkung (s. Abbildung).

### Antigestagene

Mit dem Antigestagen RU 486 war eine Unterbrechung einer bestehenden Schwangerschaft mit 10 mg/d s.c. bei 3 von 9 behandelten Tieren zu erzielen. Die Aborte erfolgten mit einer 4- bis 6tägigen Latenz vom Behandlungsbeginn (s. Abbildung).

### Antigestagen/Progesteronsynthesehemmer-Kombination

Die Kombination von marginal wirksamen Antigestagendosen (10,0 mg RU 486/d s.c.) mit einer unwirksamen Epostandosis von 30 mg/d p.o. führte zu einer 100 %igen Abortrate und zu einem weit schnelleren Auftreten der Aborte. Die Latenzzeit ist auf maximal 2 Tage verkürzt worden (s. Abbildung).

## Patentansprüche

1. Arzneimittel, dadurch gekennzeichnet, daß es einen Progesteronsynthesehemmer (ProH) vom Typ des Trilostans oder Epostans und ein Antigestagen (AG) enthält.

2. Arzneimittel gemäß Anspruch 1, dadurch gekennzeichnet, daß Progesteronsynthesehemmer und Antigestagen in einem Gewichtsverhältnis von 1:40 bis 60:1 stehen.

3. Arzneimittel gemäß Anspruch 1, dadurch gekennzeichnet, daß Progesteronsynthesehemmer und Antigestagen in getrennten Dosiseinheiten vorliegen.

4. Arzneimittel gemäß Anspruch 1, dadurch gekennzeichnet, daß Progesteronsynthesehemmer und Antigestagen in einer gemeinsamen Dosiseinheit vorliegen.

5. Arzneimittel gemäß Anspruch 1, dadurch gekennzeichnet, daß eine Progesteronsynthesehemmer-Dosiseinheit 5 bis 600 mg Epostan (4α,5-Epoxy-3,17β-dihydroxy-4β,17α,dimethyl-5α-androst-2-en-2-carbonitril) oder eine biologoisch äquivalente Menge eines anderen Progesteronsynthesehemmers vom Typ des Trilostans oder Epostans enthält.

6. Arzneimittel gemäß Anspruch 1, dadurch gekennzeichnet, daß eine AG-Dosiseinheit 5 bis 200 mg 11β-[(4-N,N-Dimethylamino)phenyl]-17-β-hydroxy-17α-propinyl-4,9(10)-estradien-3-on oder eine biologisch äquivalente Menge eines anderen Antigestagens enthält.

7. Verwendung von Progesteronsynthesehemmern vom Typ des Trilostans oder Epostans und Antigestagenen zur Herstellung von Arzneimitteln zur getrennten oder gemeinsamen, gleichzeitigen oder zeitlich abgestuften Anwendung zum Abbruch der Gravidität, zur Geburtseinleitung, zur Behandlung hormonabhängiger Tumore, der Endometriose und der Dysmenorrhoe.

8. Verwendung nach Anspruch 7, dadurch gekennzeichnet, daß Progesteronsynthesehemmer und Antigestagen in einem Gewichtsverhältnis von 1:40 bis 60:1 stehen.

9. Verwendung von 5 bis 600 mg Epostan als Progesteronsynthesehemmer oder einer biologisch äquivalenten Menge eines anderen Progesteronsynthesehemmers vom Typ des Trilostans oder Epostans gemaß Anspruch 7.

## Claims

1. Medicament, characterized in that it contains a progesterone synthesis inhibitor (ProH) of the trilostane or epostane type and an antigestagen (AG).

2. Medicament according to claim 1, characterized in that the progesterone synthesis inhibitor and the antigestagen are in a weight ratio of from 1:40 to 60:1.

3. Medicament according to claim 1, characterized in that the progesterone synthesis inhibitor and the antigestagen are in separate dosage units.

4. Medicament according to claim 1, characterized in that the progesterone synthesis inhibitor and the antigestagen are in a combined dosage unit.

5. Medicament according to claim 1, characterized in that a progesterone synthesis inhibitor dosage unit contains from 5 to 600 mg of epostane (4α,5-Epoxy-3,17β-dihydroxy-4β,17α,dimethyl-5α-androst-2-en-2-carbonitrile) or a biologically equivalent amount of a different progezterone synthesis inhibitor of the trilostane or epostane type.

6. Medicament according to claim 1, characterized in that an AG dosage unit contains from 5 to 200 mg of 11β-[(4-N,N-Dimethylamino)-phenyl]-17-β-hydroxy-17α-propinyl-4,9(10)-estradien-3-one or a biologically equivalent amount of a different antigestagen.

7. Use of progesterone synthesis inhibitors of the trilostane or epostane type and antigeztagens for the preparation of medicaments for separate or combined, simultaneous or staggered administration to terminate pregnancy, induce labour and treat hormone-dependent tumours, endometriosis and dysmenorrhoea.

8. Use according to claim 7, characterized in that the progesterone synthesis inhibitor and the antigestagen are in a weight ratio of from 1:40 to 60:1.

9. Use of from 5 to 600 mg of epostane as progesterone synthesisinhibitor or a biologically equivalent amount of a different progesterone synthesis inhibitor of the trilostane or epostane type according to claim 7.

## Revendications

1. Médicament caractérisé en ce qu'il comprend un inhibiteur de synthèse de la progestérone (ProH) du genre du trilostane ou de l'épostane, avec un antigestagène (AG).

2. Médicament selon la revendication 1 caractérisé en ce que l'inhibiteur de progestérone et l'antigestagène sont dans un rapport pondéral compris entre 1:40 et 60:1.

3. Médicament selon la revendication 1 caractérisé en ce que l'inhibiteur de progestérone et l'antigestagène sont placés séparément en unités de doses.

4. Médicament selon la revendication 1 caractérisé en ce que l'inhibiteur de progestérone et l'antigestagène sont dans une unité de dose commune.

5. Médicament selon la revendication 1 caractérisé en ce qu'une unité de dose de l'inhibiteur de progestérone contient de 5 à 600 mg d'épostane (4α,5-Epoxy-3,17β-dimethyl-5α-androst-2-ène-2-carbonitrile) ou une quantité biologiquement équivalente d'un astre inhibiteur de progestérone du type du trilostane ou de l'épostane.

6. Médicament selon la revendication 1 caractérisé en ce qu'une unité de dose de l'antigestagène contient de 5 à 200 mg de 11β-[(4-N,N-Dimethylamino)-phényl]-17-β-hydroxy-17α-propynyl-4,9(10)-estradièn-3-one ou une qantité biologiquement équivalente d'un autre antigestagène.

7. L'emploi d'inhibiteurs de synthèse de la progestérone du type du trilostane ou de l'épostane avec des antigestagènes pour en préparer des médicaments en vue d'administrations séparées ou communes, simultanées ou à intervalles de temps, pour interrompre une grossesse, conduire un accouchement et une naissance, ou pour le traitement de tumeurs hormonodépendantes, de l'endométriose ou de la dysménorrhée.

8. Emploi selon la revendication 7, caractérisé en ce l'inhibiteur de progestérone et l'antigestagène sont donnés dans un rapport pondéral compris entre 1:40 et 60:1.

9. Emploi de 5 à 600 mg d'épostane comme inhibiteur de synthèse de la progestérone, ou d'une dose biologiquement équivalente d'un autre inhibiteur de progestérone du type du trilostane ou de l'épostane, selon la revendication 7.
